# EUROPEAN PATENT APPLICATION

(11) **EP 4 741 485 A1**
(43) Date of publication of application: **13.05.2026**
(21) Application number: 24839312.6
(22) Date of filing: 17.05.2024
(51) Int. Cl.: C12M 1/00, C12N 11/00

(54) **MICROFLUIDIC CHIP**

(30) Priority: 07.07.2023 JP 2023112309
(71) Applicant: National Institute of Advanced Industrial Science and Technology, Chiyoda-ku Tokyo 100-8921 (JP)
(72) Inventor: FUJITA, Satoshi, Tsukuba-shi, Ibaraki 305-8560 (JP); ZHANG, Huiting, Tsukuba-shi, Ibaraki 305-8560 (JP); ESPULGAR, Wilfred Villariza, Tsukuba-shi, Ibaraki 305-8560 (JP); MATSUSAKI, Michiya, Tsukuba-shi, Ibaraki 305-8560 (JP)
(74) Representative: Bandpay & Greuter
(86) International application number: PCT/JP2024/018363
(87) International publication number: WO 2025/013415

(57) **Abstract**

Provided is a microfluidic chip having enhanced visibility and connection of an interface structure. The microfluidic chip includes a first substrate and a second substrate. The first substrate includes: a first groove part extending in a first direction; a second groove part, a portion of which extends in the first direction; a third groove part, a portion of which extends in the first direction; a first protrusion part; and a second protrusion part. The first groove part and the second groove part are adjacent to each other with the first protrusion part in between, in a second direction perpendicular to the first direction. The first groove part and the third groove part are adjacent to each other with the second protrusion part in between, on an opposite side in a direction in which the second groove part is adjacent to. The first groove part, the second groove part, and the third groove part each have, at opposite ends thereof, openings penetrating the first substrate. The first substrate and the second substrate are stacked in a third direction perpendicular to the first direction and to the second direction, so that the first groove part forms a first flow path, the second groove part forms a second flow path, and the third groove part forms a third flow path.

## Description

### Technical Field

The present invention relates to a microfluidic chip, in particular, to a Microphysiological System (MPS) chip.

### Background Art

In recent years, microfluidic chips formed a Microphysiological System (MPS) therein (MPS chips) are attracting attention as an organ model to replace animal testing. An MPS chip, in which an organ tissue and a lumen structure are constructed, is used for a pharmacokinetic analysis or the like by observing molecules entering and exiting at an interface.

A commonly MPS chip in one form has a structure in which two or three flow path structures are connected at a central part (see Patent Literature 1, etc.). In the case of two flow paths, an organ is created in one of the flow paths, and a lumen structure is created in the other flow path. In the case of three flow paths, an organ is created in a central channel, and a lumen structure is created on either side thereof.

In Patent Literature 1, an MPS chip is formed by providing three flow paths as a relief pattern on a film surface and joining a cover therewith. In Patent Literature 1, cell culture is carried out by using the produced MPS chip so as to observe mass transfer in a Rhodamine perfusion.

### Citation List

### Patent Literature

Patent Literature 1: Japanese Translation of PCT International Application Publication No. 2021-513856

### Summary of Invention

### Technical Problem

However, in the chip disclosed in Patent Literature 1, because the flow paths are provided on the side of a lower layer film, there is a possibility that the relief pattern formed on the lower layer may reduce visibility of the structures when the observation is made from the lower surface of the chip, by using an inverted microscope, an Optical Coherence Tomography (OCT), or the like. Further, when a fence partitioning the flow paths is too high, contact surfaces between two flow paths become small, leading to difficulty of connections at the interface.

In view of the above, an object of the present invention is to provide a microfluidic chip having enhanced visibility and connection of an interface structure.

### Solution to Problem

To solve the abovementioned problem, a microfluidic chip of the present invention includes a first substrate and a second substrate. The first substrate includes: a first groove part extending in a first direction; a second groove part, a portion of which extends in the first direction; a third groove part, a portion of which extends in the first direction; a first protrusion part; and a second protrusion part. The first groove part and the second groove part are adjacent to each other with the first protrusion part in between in a second direction perpendicular to the first direction. The first groove part and the third groove part are adjacent to each other with the second protrusion part in between, on an opposite side in a direction in which the second groove part is adjacent to. The first groove part, the second groove part, and the third groove part each have, at opposite ends thereof, openings penetrating the first substrate. The first substrate and the second substrate are stacked in a third direction perpendicular to the first direction and to the second direction, so that the first groove part forms a first flow path, the second groove part forms a second flow path, and the third groove part forms a third flow path.

Further, another embodiment of the present invention relates to a microphysiological chip manufacturing method including: a step of installing a microfluidic chip according to the present embodiment in such a manner that the third direction is a direction opposite from a gravity direction, and the second substrate is on a lower side; a step of injecting, into the first flow path, and gelling a fluid containing at least one type of cells and a gelling agent; and a step of injecting a culture medium solution into the second flow path and the third flow path and carrying out cell culture.

### Advantageous Effects of Invention

The present invention is able to provide the microfluidic chip having enhanced visibility and connection of an interface structure.

### Brief Description of Drawings

[Figure 1] Figure 1(a) is an exterior view of a microfluidic chip according to an embodiment of the present invention; Figure 1(b) is a sectional view taken at line Ib-Ib in Figure 1(a); and Figure 1(c) is a sectional view taken at line Ic-Ic in Figure 1(a).
[Figure 2] Figure 2(a) is a top view of a microfluidic chip according to Example 1 of the present invention; Figure 2(b) is a top view of the vicinity of a center part of Figure 2(a); and Figure 2(c) is a sectional view taken at line IIc-IIc in Figure 2(a).
[Figure 3] Figure 3(a) is a top view of a microfluidic chip according to Example 2 of the present invention; Figure 3(b) is a top view of the vicinity of a center part of Figure 3(a); and Figure 3(c) is a sectional view taken at line IIIc-IIIc in Figure 3(a).
[Figure 4] Figure 4(a) is a top view of a microfluidic chip according to Example 3 of the present invention; Figure 4(b) is a top view of the vicinity of a center part of Figure 4(a); and Figure 4(c) is a sectional view taken at line IVc-IVc in Figure 4(a).
[Figure 5] Figure 5(a) is a top view of a microfluidic chip according to Example 4 of the present invention; Figure 5(b) is a top view of the vicinity of a center part of Figure 5(a); and Figure 5(c) is a sectional view taken at line Vc-Vc in Figure 5(a).

### Description of Embodiments

Embodiments of the present invention will be explained in detail below. The present invention is not limited to these examples.

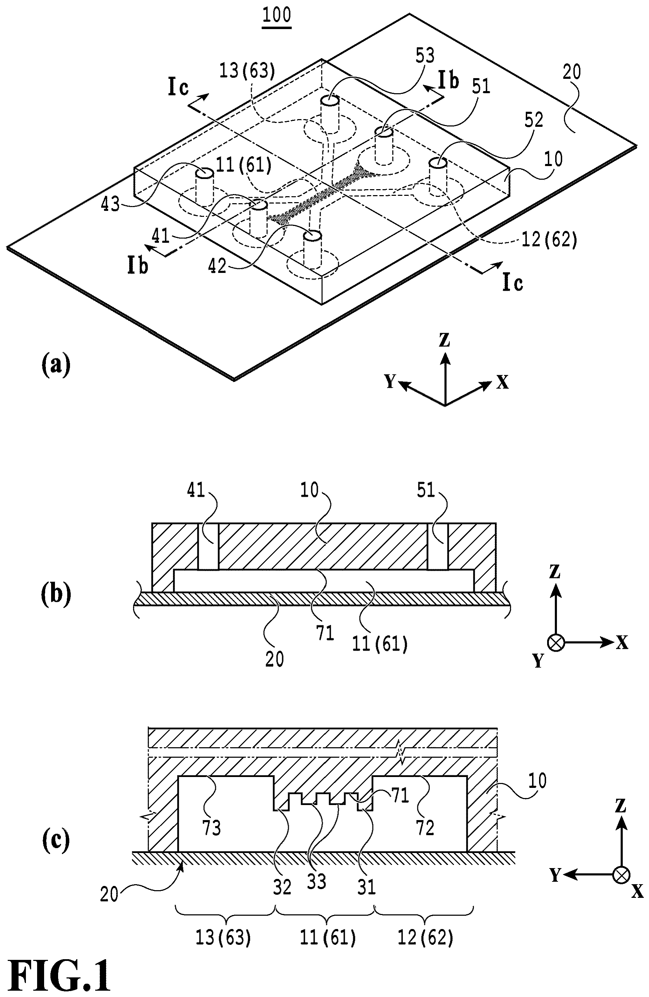

Figure 1(a) is an exterior view of a microfluidic chip 100 according to an embodiment of the present invention. As shown in Figure 1(a), the microfluidic chip 100 includes a first substrate 10 and a second substrate 20.

The first substrate 10 includes a first groove part 11 extending in a first direction; a second groove part 12, a portion of which extends in the first direction; and a third groove part 13, a portion of which extends in the first direction. Via a first protrusion part 31, the first groove part 11 and the second groove part 12 are adjacent to each other in a second direction perpendicular to the first direction. Via a second protrusion part 32, the first groove part 11 and the third groove part 13 are adjacent to each other on the opposite side in the direction in which the second groove part 12 is adjacent to (see Figures 1(a) and 1(c)).

Further, the first groove part 11, the second groove part 12, and the third groove part 13 each have openings (41, 42, 43, and 51, 52, 53) at opposite ends thereof. The openings penetrate the first substrate 10 as shown in Figure 1(b).

As shown in Figure 1(b) and Figure 1(c), the first substrate 10 and the second substrate 20 are stacked in a third direction perpendicular to the first direction and to the second direction and have flow paths (61, 62, and 63) formed inside.

In the present disclosure, as directional axes for explaining structures and the like of the microfluidic chip 100, the first direction will be referred to as an X axis, the second direction will be referred to as a Y axis, and the third direction will be referred to as a Z axis. The X axis and the Y axis are orthogonal to each other on a horizontal plane, and the flow paths (61, 62, and 63) are formed on the plane. During use, the third direction (the Z axis) of the microfluidic chip 100 is oriented in the direction opposite from the gravity direction. Constituent elements will be described in detail below.

### 1. The first substrate

The first substrate 10 includes, on mutually the same plane, the first groove part 11 extending in the first direction (the X axis direction); the second groove part 12, a portion of which extends in the first direction; and the third groove part 13, a portion of which extends in the first direction. As explained later, on the first substrate 10, the second substrate 20 is stacked in the third direction (the Z axis direction) so as to cover the groove parts (11, 12, and 13), and the microfluidic chip 100 is thus formed. During use, the third direction is oriented in the direction opposite from the gravity direction, so that the microfluidic chip 100 has stacked therein the second substrate 20 as a lower layer, and the first substrate 10 as an upper layer.

As for the first groove part 11, the second groove part 12, and the third groove part 13, when the first substrate 10 and the second substrate 20 are stacked together, the first groove part 11 forms a first flow path 61, the second groove part 12 forms a second flow path 62, and the third groove part 13 forms a third flow path 63.

The distance between surfaces (ceiling surfaces 71, 72, and 73) of the flow paths (61, 62, and 63) facing the second substrate 20 and the second substrate 20, i.e., the depths of the groove parts (11, 12, and 13) in the Z axis direction may be in the range of 10 µm to 600 µm, for example, although the depths may vary depending on materials of the first substrate 10, surface treatment of the flow paths (61, 62, and 62), and physical properties of a fluid to be injected into the microfluidic chip 100. Because of a fine structure as described herein, the fluid to be injected in the microfluidic chip 100 is able to move while filling, with surface tension, the space between the ceiling surfaces (71, 72, and 73) of the flow paths and the second substrate 20.

The depths of the groove parts (11, 12, and 13) in the Z axis direction may be equal to or may be different from one another. For example, the first groove part 11 may be shallower than the second groove part 12 and the third groove part 13. On the contrary, the first groove part 11 may be deeper than the second groove part 12 and the third groove part 13. Furthermore, the second groove part 12 and the third groove part 13 may have mutually-different depths.

The first substrate 10 further includes the first protrusion part 31 and the second protrusion part 32 projecting in the Z axis direction. Via the first protrusion part 31, the first groove part 11 and the second groove part 12 are adjacent to each other in the second direction (the Y axis direction) perpendicular to the first direction. Via the second protrusion part 32, the first groove part 11 and the third groove part 13 are adjacent to each other on the opposite side in the direction in which the second groove part 12 is adjacent to (see Figures 1(a) and 1(c)).

The heights of the first protrusion part 31 and the second protrusion part 32 in the Z axis direction are smaller than the absolute value of the depth of each of the groove parts (11, 12, and 13) in the Z axis direction. In other words, in the microfluidic chip 100 in the present embodiment, there is a space in the Z axis direction between the first and the second protrusion parts 31, 32 and the second substrate 20. Thus, because contact surface between any two flow paths spreads in a planar manner, mass transfer (e.g., interactions of signal compounds, nutrient sources, enzymes, and the like between interfaces) is able to occur more smoothly at the interface of any two flow paths.

As described later, it is sufficient that the heights of the first protrusion part 31 and the second protrusion part 32 in the Z axis direction are such heights that a fluid (a gel fluid) flowing through the first flow path 61 flows without leaking from the first flow path 61. The gel fluid contains a gellable substance and generally has a viscosity higher than that of purified water. It is sufficient that the gel fluid is allowed to flow into the first flow path 61, and the gel fluid may typically be a liquid.

In the present embodiment, in the structure described above, the gel fluid is maintained, by the surface tension, between the first and the second protrusion parts 31, 32 and the second substrate 20, and is able to flow along the first flow path 61. The dimensions of the first protrusion part 31 and the second protrusion part 32 may be adjusted, as appropriate, according to materials of the first substrate 10, the surface treatment of the flow paths (61, 62, and 62), and the physical properties of the fluid to be injected into the microfluidic chip 100. The heights of the first protrusion part 31 and the second protrusion part 32 in the Z axis direction may be in the range of 4 µm to 240 µm, for example.

Further, the first protrusion part 31 and the second protrusion part 32 may each have a rail-like shape extending in the X axis direction or may each be in the shape of pillars standing in a row in the X axis direction. When the first protrusion part 31 and the second protrusion part 32 each have a rail-like shape, for example, the width thereof may be larger than 5 µm and equal to or smaller than 300 µm. In the X axis direction, the rail-like shape may extend uninterruptedly or may extend interruptedly. Further, when the first protrusion part 31 and the second protrusion part 32 are each in the shape of the pillars, a cross-section of each pillar may be circular or polygonal. When each pillar is a circular column, the circle on a cross-section may have a diameter larger than 5 µm and equal to or smaller than 300 µm.

In another embodiment, the first substrate 10 may further have a third protrusion part 33 in the first groove part 11. The third protrusion part 33 may preferably have a pillar shape. A cross-section of the pillar may be circular or may be polygonal. Also, it is desirable that the height of the third protrusion part 33 in the Z axis direction is shorter than the heights of the first protrusion part 31 and the second protrusion part 32 in the Z axis direction. With this structure, although not limited to this example, the gel fluid flowing through the first flow path 61 is inhibited from spreading in the Y axis direction by the surface tension of the gel fluid with the first protrusion part 31 and the second protrusion part 32. In addition, due to an increase caused by the third protrusion part 33 in the contact surface area between the gel fluid and the first substrate 10, it is possible to further inhibit the spreading in the Y axis direction because of an increase in shear force and friction force required in order for the gel fluid to start moving.

Further, the first groove part 11, the second groove part 12, and the third groove part 13 each have the openings (41, 42, 43, and 51, 52, 53) at the opposite ends thereof. The openings penetrate the first substrate 10 as shown in Figure 1(b). Of each pair of openings, either one may be on the entrance side and either one may be on the exit side. In the microfluidic chip 100 of the present embodiment, supposing that the opening 41 is an entrance of the first flow path 61, the fluid injected through the opening 41 goes through the first flow path 61 and moves in the X axis direction, before flowing to the opening 51 serving as an exit.

It is sufficient that the openings (41, 42, 43, and 51, 52, 53) are shaped to penetrate between the outside of the first substrate 10 and the opposite ends of the groove parts (11, 12, and 13) and may penetrate along the Z axis direction, for example. In another embodiment, the openings may penetrate diagonally with respect to the Z axis direction or in a direction perpendicular to the Z axis direction. Further, the individual openings may be oriented in mutually-different directions.

Although the material used for forming the first substrate 10 is not particularly limited, it is desirable that the material is translucent for observations of cell culture processes or mass transfer inside the microfluidic chip 100. Further, it is also desirable that the material is oxygen-permeable so that the cell culture is properly carried out inside the microfluidic chip 100. For instance, examples of the material that can be used for forming the first substrate 10 include polydimethylsiloxane (PDMS), polystyrene (PS), cycloolefin polymers, acrylic resin, and silicone rubber.

### 2. The second substrate

The second substrate 20 is stacked on the first substrate 10 in the third direction (in the Z axis direction) so as to cover the groove parts (11, 12, and 13) of the first substrate 10, and the microfluidic chip 100 is thus formed. During use, the third direction is oriented in the direction opposite from the gravity direction, so that the second layer 20 serves as a lower layer, while the first layer 10 is stacked on the second layer 20.

It is sufficient that the second substrate 20 has a flat shape and may have a platelike shape or a film-like shape. The smaller the thickness of the second substrate 20 is, the more closely a focal point depth can be brought to an observed subject, for the observation from the second substrate 20 side.

Although the material used for forming the second substrate 20 is not particularly limited, it is desirable that the material is translucent for observations of cell culture processes or mass transfer inside the microfluidic chip 100. Further, it is also desirable that the material is oxygen-permeable so that the cell culture is properly carried out inside the microfluidic chip 100. For instance, examples of the material that can be used for forming the second substrate 20 include glass, polydimethylsiloxane (PDMS), polystyrene (PS), cycloolefin polymers, acrylic resin, and silicone rubber. When observation is to be made by using Raman spectroscopy or the like, it is possible to use quartz, calcium fluoride, or the like having a wider transmission wavelength range. When a film-like material is used, it is possible to use an OPS (polystyrene) film, a polyethylene terephthalate (PET) film, a COC (cycloolefin) film, or the like. Further, the second substrate 20 may be subjected to surface modification by optical excitation, plasma processing, a silylating agent, or the like.

### 3. The Microfluidic Chip

It is possible to form the microfluidic chip 100 by stacking and pasting together the first substrate 10 and the second substrate 20 that have been processed.

For processing the first substrate 10, it is possible to use a microfabrication technique known in the relevant field such as photolithography or nanoimprint. Further, the groove parts and the openings that have been formed may be subjected to surface modification by optical excitation, plasma processing, or the like.

In an embodiment, the microfluidic chip 100 may be produced by performing the following procedure:
(1) A microchannel mold for the first substrate 10 is formed by performing soft lithography on a silicon wafer while using SU-8 (manufactured by MicroChem Corp.).
(2) By using the microchannel mold, the first substrate 10 is produced in which the groove parts are formed with PDMS.
(3) Through holes are formed in the formed first substrate 10.
(4) The first substrate 10 in which the groove parts and the through holes have been formed is pasted onto a glass sheet serving as the second substrate 20.

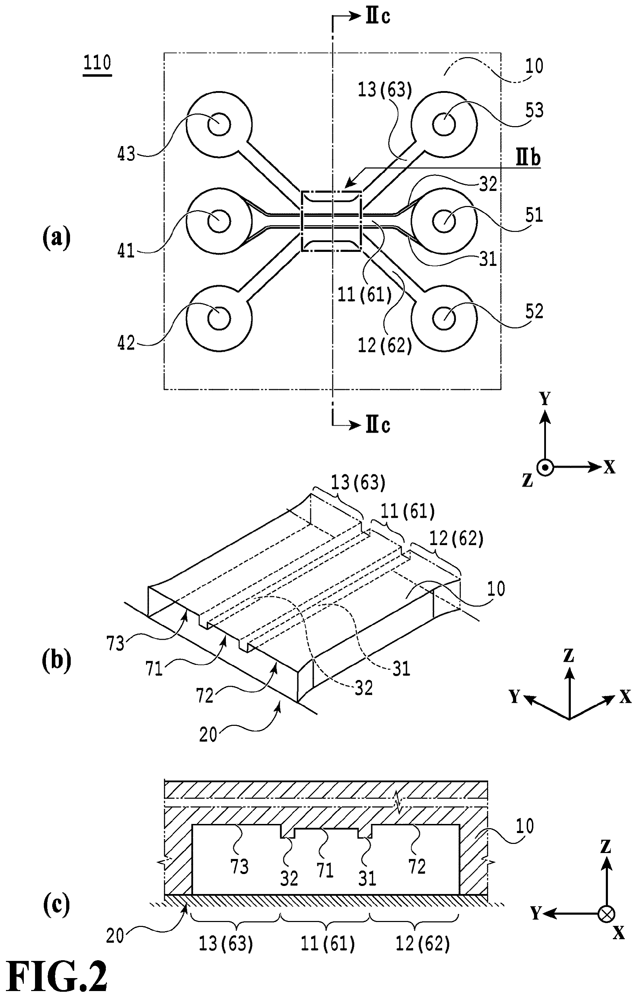

Figures 2 to 5 show microfluidic chips (110, 120, 130, and 140) according to Examples 1 to 4. In these Examples, the first substrates 10 are formed by using PDMS, while glass sheets are used as the second substrate 20; however, possible embodiments are not limited to these materials or the structures.

Figure 2 shows the microfluidic chip 110 according to Example 1. In the present embodiment, in the microfluidic chip 110, the first protrusion part 31 and the second protrusion part 32 each have a rail-like shape extending in the X axis direction. In this structure, when a gel fluid is injected into the microfluidic chip 110, the gel fluid is maintained between the first and the second protrusion parts 31, 32 and the second substrate 20 by surface tension and is thus able to flow along the first flow path 61.

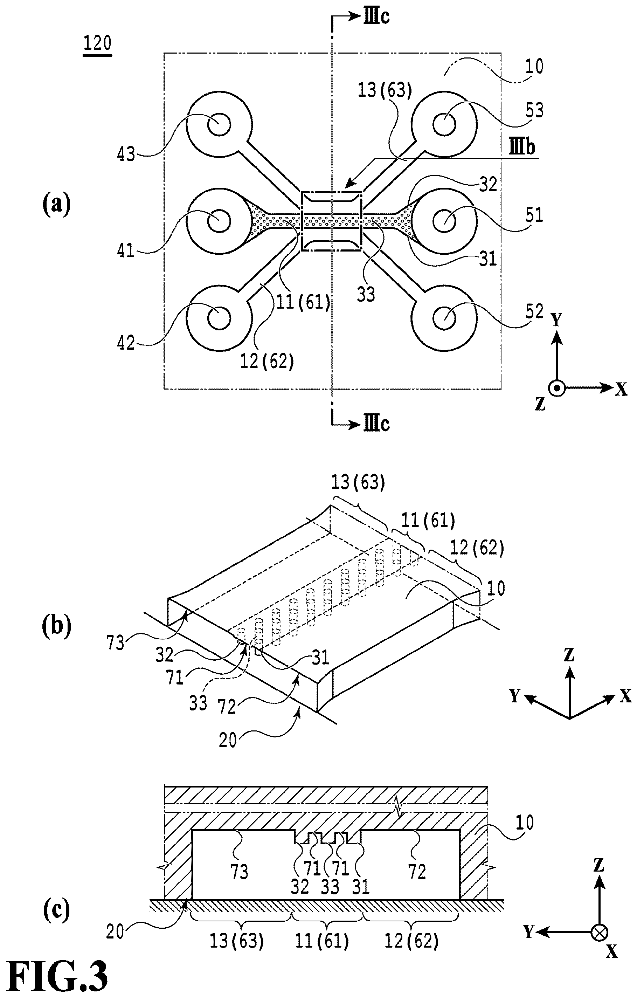

Figure 3 shows the microfluidic chip 120 according to Example 2. In the present embodiment, in the microfluidic chip 120, the first protrusion part 31 and the second protrusion part 32 are each in the shape of circular columnar pillars. Further, in the first groove part 11 provided between the first protrusion part 31 and the second protrusion part 32, the third protrusion part 33 having the same shape as the shapes of the first protrusion part 31 and the second protrusion part 32 is formed. Accordingly, when a gel fluid is injected into the microfluidic chip 120, the gel fluid flowing through the first flow path 61 is inhibited from spreading in the Y axis direction, by the surface tension of the gel fluid with the first protrusion part 31 and the second protrusion part 32. In addition, it is also possible to inhibit the spreading in the Y axis direction because of an increase in shear force and friction force with respect to the third protrusion part 33.

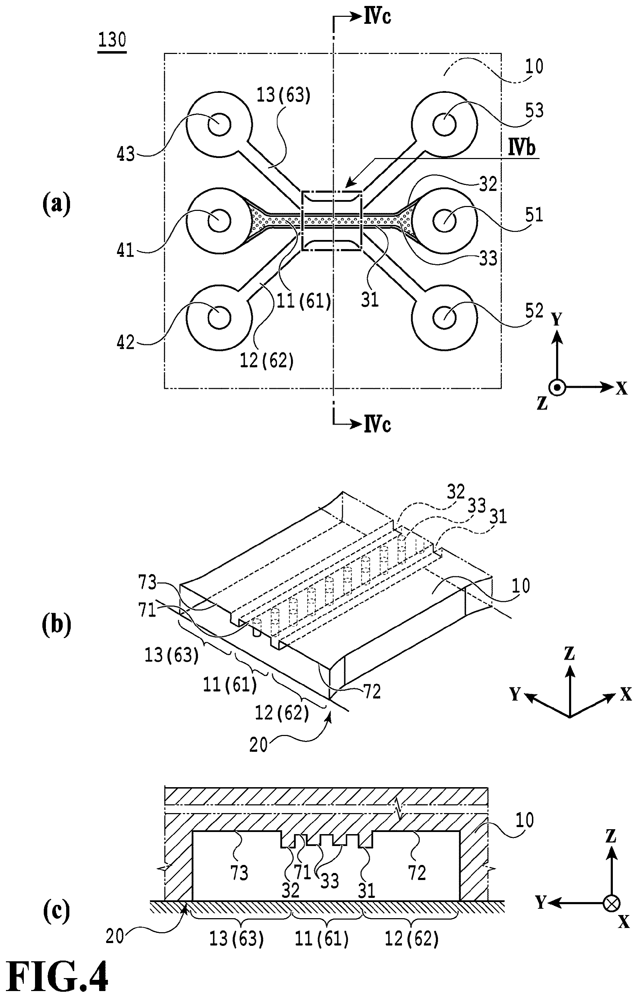

Figure 4 shows the microfluidic chip 130 according to Example 3. In the present embodiment, the microfluidic chip 130 has the third protrusion part 33 in the first groove part 11 provided between the first protrusion part 31 and the second protrusion part 32 each having a rail-like shape and extending in the X axis direction. The third protrusion part 33 is in the shape of circular columnar pillars having a height that is shorter than the heights of the first protrusion part 31 and the second protrusion part 32 in the Z axis direction. In this structure, when a gel fluid is injected into the microfluidic chip 130, the gel fluid flowing through the first flow path 61 is inhibited from spreading in the Y axis direction by the surface tension of the gel fluid with the first protrusion part 31 and the second protrusion part 32. In addition, the gel fluid is able to flow, in the X axis direction, through the central part of the first groove part 11 having the third protrusion part 33, while being inhibited from spreading in the Y axis direction because of an increase in the shear force and the friction force with respect to the third protrusion part 33.

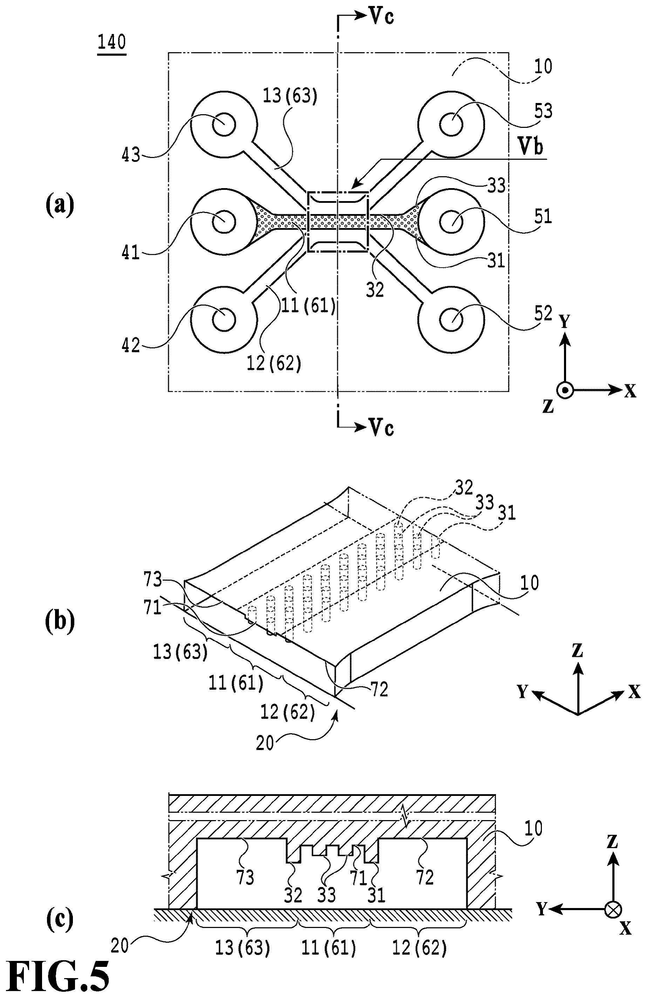

Figure 5 shows the microfluidic chip 140 according to Example 4. In the present embodiment, the microfluidic chip 140 has the third protrusion part 33 in the first groove part 11 provided between the first protrusion part 31 and the second protrusion part 32 each in the shape of circular columnar pillars standing in a row in the X axis direction. The third protrusion part 33 is in the shape of pillars having a height that is shorter than the heights of the first protrusion part 31 and the second protrusion part 32 in the Z axis direction. In this structure, when a gel fluid is injected into the microfluidic chip 140, the gel fluid flowing through the first flow path 61 is inhibited from spreading in the Y axis direction by the surface tension of the gel fluid with the first protrusion part 31 and the second protrusion part 32. In addition, the gel fluid is able to flow, in the X axis direction, through the central part of the first groove part 11 having the third protrusion part 33, while being inhibited from spreading in the Y axis direction because of an increase in the shear force and the friction force with respect to the third protrusion part 33.

### 4. A microphysiological system using a microfluidic chip

As Example 5, a Blood Brain Barrier (BBB) chip was formed by using the microfluidic chip 130 in Example 3, and an interface state and mass transfer between channels were observed.

The structure of the microfluidic chip 130 that was used is presented below.

**[Table 1]**

| (Table 1) Structure of Microfluidic Chip 130 | | |
|---|---|---|
| First Substrate 10 | Material | PDMS |
| | Substrate Size | 21 mm × 21 mm × 5 mm |
| | First Groove Part 11 Depth in Z axis Direction | 140 µm |
| | First Groove Part 11 Length in Y axis Direction | 600 µm |
| | Second Groove Part 12 Depth in Z axis Direction | 165 µm |
| | Second Groove Part 12 Length in Y axis Direction | 600 µm |
| | Third Groove Part 13 Depth in Z axis Direction | 165 µm |
| | Third Groove Part 13 Length in Y axis Direction | 600 µm |
| | First Protrusion Part 31 Height in Z axis Direction | 65 µm |
| | First Protrusion Part 31 Length in Y axis Direction | 112 µm |
| | Second Protrusion Part 32 Height in Z axis Direction | 65 µm |
| | Second Protrusion Part 32 Length in Y axis Direction | 112 µm |
| | Third Protrusion Part 33 Height in Z axis Direction | 65 µm |
| | Third Protrusion Part 33 Pillar Diameter | 60 µm |
| Second Substrate 20 | Material | Glass |
| | Substrate Size | 24 mm × 36 mm × 0.17 mm |

By using the microfluidic chip 130, the BBB chip was produced as follows:
(1) The first substrate 10 and the second substrate 20 of the microfluidic chip 130 were processed with plasma.
(2) The microfluidic chip 130 was installed so that the Z axis direction was oriented in the direction opposite from the gravity direction while the second substrate 20 served as the lower surface and, for a joining purpose, was incubated on a 65°C hot plate overnight or longer.
(3) A solution (0.1 µg/ml) of Poly-D-Lysine (MP Biomedicals, Inc. USA) was added to the inside of the flow paths, and the workpiece was left in an incubator at 37°C overnight or longer.
(4) The inside of the flow paths was cleaned with ultrapure water six times or more and was dried.
(5) A gel fluid created by mixing fibrin gel with cerebral vascular endothelial cells, an astrocyte, and a pericyte was introduced into the first flow path 61 through the opening 41 of the microfluidic chip 130, and was hardened, so as to form a tissue channel.
(6) After the gel fluid was gelled, a culture medium for a cell culture purpose containing vascular endothelial cells was introduced into the second flow path 62 through the opening 42, so as to form a blood vessel channel. Further, a culture medium for a cell culture purpose was introduced into the third flow path 63 through the opening 43, so as to form a brain channel. The culture medium for the cell culture purpose introduced into the second flow path 62 and the culture medium for the cell culture purpose introduced into the third flow path 63 may each appropriately be selected in accordance with the cells to be cultured. Accordingly, the culture medium for the cell culture purpose introduced into the second flow path 62 and the culture medium for the cell culture purpose introduced into the third flow path 63 may be different from each other or may be the same as each other.
(7) While the culture media in (6) were each circulated, cell culture was carried out for seven days.
(8) A BBB structure was formed in the microfluidic chip 100. By using an inverted microscope, the BBB structure and mass transfer were observed from the second substrate 20 side.

In the BBB structure formed in this manner, selective movements of specific chemical substances to the brain channel were observed, and it was confirmed that the structure was usable as a microphysiological system. It is considered that the reason was, although not limited to the theory described here, that it was possible to have the gel fluid maintained in the first flow path 61 by the first protrusion part 31, the second protrusion part 32, and the third protrusion part 33, and it was therefore possible to locally carry out the cell culture and to promote tight junctions between the cells.

Further, in the present embodiment between the channels, because the second substrate 20 has a flat structure, there is no structure in the microfluidic chip 100 that obstructs the observation of the interface structure. It is therefore possible to make the observation with excellent visibility. For example, it is possible to observe angiogenesis growing from the interface. Furthermore, in the present embodiment, because there is a space between the first and the second protrusion parts 31, 32 and the second substrate 20, the contact surfaces between the tissue channel at the center and lumen channels on either side thereof spread in a planar manner, mass transfer is able to occur more smoothly at the interfaces between the channels.

While the BBB structure was formed within the microfluidic chip 100 in the above Examples, the present invention may also be applicable to modelization of other microphysiological systems such as a mass transfer model involving a membrane tissue, like a liver, small intestine, or large intestine model.

Although various embodiments and modification examples have been explained above, the present invention is not limited to the descriptions thereof. Other modes that are conceivable within the scope of technical concepts of the present invention are also included in the scope of the present invention.

Furthermore, one or more of the embodiments and the modification examples described above may be combined as appropriate.

### Reference Signs List

10: first substrate
11: first groove part
12: second groove part
13: third groove part
20: second substrate
31: first protrusion part
32: second protrusion part
33: third protrusion part
41: opening
42: opening
43: opening
51: opening
52: opening
53: opening
61: first flow path
62: second flow path
63: third flow path
71: ceiling surface
72: ceiling surface
73: ceiling surface
100: microfluidic chip
110: microfluidic chip
120: microfluidic chip
130: microfluidic chip
140: microfluidic chip

## Claims

1. A microfluidic chip comprising a first substrate and a second substrate, wherein
the first substrate includes:
a first groove part extending in a first direction;
a second groove part, a portion of which extends in the first direction;
a third groove part, a portion of which extends in the first direction;
a first protrusion part; and
a second protrusion part,
the first groove part and the second groove part are adjacent to each other with the first protrusion part in between in a second direction perpendicular to the first direction,
the first groove part and the third groove part are adjacent to each other with the second protrusion part in between, on an opposite side in a direction in which the second groove part is adjacent to,
the first groove part, the second groove part, and the third groove part each have, at opposite ends thereof, openings penetrating the first substrate, and
the first substrate and the second substrate are stacked in a third direction perpendicular to the first direction and to the second direction, so that the first groove part forms a first flow path, the second groove part forms a second flow path, and the third groove part forms a third flow path.

2. The microfluidic chip according to claim 1, wherein
there is a space between the first and the second protrusion parts and the second substrate.

3. The microfluidic chip according to claim 1, wherein
the first groove part further has a third protrusion part.

4. The microfluidic chip according to claim 3, wherein
a height of the third protrusion part is shorter than heights of the first protrusion part and the second protrusion part.

5. The microfluidic chip according to claim 1, wherein
the first protrusion part and the second protrusion part each have a rail-like shape extending in the first direction.

6. The microfluidic chip according to claim 1, wherein
the first protrusion part and the second protrusion part are each in a shape of a plurality of pillars standing in a row in the first direction, the plurality of pillars each having a circular or polygonal cross-section perpendicular to the third direction.

7. The microfluidic chip according to claim 1, wherein
the second substrate is translucent.

8. The microfluidic chip according to claim 1, wherein
the second substrate is oxygen-permeable.

9. The microfluidic chip according to claim 1, wherein
the first substrate is translucent.

10. The microfluidic chip according to claim 1, wherein
the first substrate is oxygen-permeable.

11. A microphysiological chip manufacturing method comprising:
a step of installing the microfluidic chip according to any one of claims 1 to 10 in such a manner that the third direction is a direction opposite from a gravity direction, and the second substrate is on a lower side;
a step of injecting, into the first flow path, and gelling a fluid containing at least one type of cells and a gelling agent; and
a step of injecting a culture medium solution into the second flow path and the third flow path and carrying out cell culture.
